# EUROPEAN PATENT SPECIFICATION

(11) **EP 2 346 401 B1**
(45) Date of publication and mention of the grant of the patent: **22.05.2013**
(21) Application number: 08813350.9
(22) Date of filing: 30.09.2008
(51) Int. Cl.: A61B 5/053, A61N 1/37, A61B 5/0452, A61N 1/365, A61B 5/0472

(54) **HEART FAILURE DETECTOR**
DETEKTOR FÜR HERZVERSAGEN
DÉTECTEUR D INSUFFISANCE CARDIAQUE

(43) Date of publication of application: 27.07.2011
(73) Proprietor: St. Jude Medical AB, 175 84 Järfälla (SE)
(72) Inventor: HOLMSTRÖM, Nils, S-175 57 Järfälla (SE); BROOMÉ, Michael, S-178 38 Ekerö (SE); BLOMQVIST, Andreas, S-163 43 Spånga (SE)
(74) Representative: Sjölander, Henrik
(86) International application number: PCT/SE2008/000537
(87) International publication number: WO 2010/039063

(56) References cited:
- WO-A1-2006/135290
- US-A- 5 029 585
- US-A- 5 137 019
- US-A1- 2002 115 939
- US-A1- 2003 074 029
- US-A1- 2005 215 914
- US-A1- 2005 216 067
- US-A1- 2007 005 114
- US-A1- 2007 066 905
- US-A1- 2008 114 410

## Description

### Technical field

The present invention generally relates to implantable medical devices, such as pacemakers or implantable cardioverter/defibrillators (ICDs), and in particular to techniques for detecting and predicting changes of heart conditions, such as heart failure, at an early stage within a patient in which a medical device is implanted.

### Background of the invention

Heart failure is a debilitating disease in which abnormal function of the heart may lead to inadequate blood flow to fulfil the needs of tissues and organs of the body or increased filling pressures. Typically, the heart loses propulsive power because the cardiac muscle loses capacity to stretch and contract. Often, the ventricles do not adequately eject or fill with blood between heartbeats and the valves regulating blood flow become leaky, allowing regurgitation or back-flow of blood. The impairment of arterial circulation deprives vital organs of oxygen and nutrients. Fatigue, weakness and the inability to carry out daily tasks may result. Not all heart failure patients suffer debilitating symptoms immediately and some may live actively for years. Yet, with few exceptions, the disease is relentlessly progressive. As heart failure progresses, it tends to be increasingly difficult to manage. A particularly severe form of heart failure is congestive heart failure (CHF) wherein the weak pumping of the heart leads to build-up of fluid in the lungs and other organs and tissues.

New medical and device-based therapies for heart failure improve survival and reduce hospitalization rates. However, total hospitalization continues to rise with the growing prevalence of heart failure. In fact, only in Europe approximately 14 million people suffer from heart failure and about 3.6 million new cases are diagnosed every year. The 5 year mortality is approximately 50%. Consequently, treating HF poses a huge stress on all European countries as well as USA's economic systems and, for example, in United States heart failure is one of the most costly diseases in healthcare budgets, with 70 % of the expenses going to the treatment of acute heart failure de-compensation.

Patients with HF eventually experience episodes of HF exacerbation requiring some kind of intervention - in some cases even hospitalization. These exacerbations may be triggered by a number of factors such as non-compliance to drugs, development of AF, changes in diet or alcohol intake, influenza etc., but may also be idiopathic. By predicting these exacerbations or more closely following the patient's HF status, early intervention is possible which may prevent the need for costly hospitalization. This also improves patient care, and in the long run, may even improve survival. Although regular monitoring of heart failure patients is recommended in management programs, none of these measures has shown conclusive impact on heart failure morbidity.

Incipient CHF is often present without the patient knowing it. An indicator of incipient CHF would therefore be of great value since treatment by addition of drugs or electrical stimulation therapy could then be introduced at an early stage of CHF to slow down the progression of CHF. This would prolong the survival of the patient. Such an indicator could also be used to alert the patient or the physician about new conditions, so that appropriate measures can be taken to avoid hospitalization. The first signs of CHF can be observed in the left atrium of the heart by monitoring its mechanical behaviour, such as volume and pressure changes. If the pumping ability of the left ventricle is reduced, the volume of the left atrium will increase due to excessive filling of blood.

Thus, as mentioned above, symptoms leading to heart failure hospitalization usually occur late in the course of de-compensation. Therefore, a reliable means of chronic fluid status monitoring in HF patients is needed to detect early de-compensation when appropriate intervention is possible.

Both literature and clinical and pre-clinical work have shown that impedance, both intracardiac and intrathoracic, decrease as HF progresses and drops significantly prior to an acute HF exacerbation. For example, US 2006/0235324 discloses a congestive heart failure monitor that measures the impedance over the left atrium, which reflects volume changes in the left atrium. Specifically, the mean value of the impedance and the quotient between the maximum value and the minimum value of the impedance during a cardiac cycle are monitored to detect CHF.

US 2003/0074029 discloses an implantable stimulator that measures a group of heart failure parameters indicative of the state of heart failure employing EGM signals, measures of blood pressure and measures of heart chamber volume. The heart failure parameters include relaxation or contraction time constant tau, mechanical restitution, recirculation fraction and end-systolic elastance.

### Summary of the invention

Thus, an object of the present invention is to provide an improved medical device and method for such a device for detecting incipient congestive heart failure.

This and other objects of the present invention are achieved by means of an implantable medical device having the features defined in the independent claims. Embodiments of the invention are characterized by the dependent claims.

The present invention is based on the insight that there is a high positive correlation between the left atrial pressure and the right ventricular pressure, particularly, at volume overload. It is well documented that patients with congestive heart failure may develop elevated systemic venous pressure indicated by e.g. jugular vein stasis. This is a direct consequence of an increased left atrial blood pressure. Since the right side of the heart and in particular the right ventricle has a higher compliance than the left side, the increased left atrial pressure will swiftly propagate through the pulmonary vessels to the right ventricle and atrium. Thus, by measuring the pressure or volume change in the right ventricle (and/or right atrium) an early and accurate indication of incipient heart failure can be achieved. The inventors have further identified a negative correlation between impedance signals measured between the right atrium and the right ventricle and the end-systolic volume of the right ventricle and a negative correlation between the measured impedance signal and the right ventricular pressure. Moreover, the inventors have also observed that the peak to peak impedance value decreases with increased left atrial pressure, which is a result of a dilatation of the right atrium and right ventricle due to increased pressure load where especially the end-systolic volume is increased. Consequently, the inventors have come to the conclusion that the impedance measured in the right ventricle (and/or right atrium) is a good parameter for detection of right ventricle dilatation and right atrium dilatation at congestive heart failure, and that this parameter in combination with the peak to peak value of the impedance provides an even more accurate indicator. This increased dilatation of the right ventricle and right atrium is a direct consequence of an increased left atrial pressure, which is well known to be an early indicator of left heart failure. This increased dilatation of the right ventricle and right atrium is caused by the differences in tissue composition between the right side and the left side of the heart. The wall of the left ventricle is thick and can thus withstand high pressure without significant volume changes, while the right ventricle, as mentioned above, is compliant to be able to pump varying volumes with low pressures. This means that a pressure increase in the left side of the heart will be to some extent "transmitted backwards" to the right heart resulting in an amplified volume change.

According to the present invention, the impedance processing unit is adapted to receive the impedance signals from the impedance measuring unit and to process the impedance signals to determine a first impedance parameter substantially reflecting a volume of the right ventricle, or, specifically, an end-systolic right ventricle volume. The impedance signals may be synchronized with ECG/IEGM signals in order to locate the end of the contraction phase in the cardiac cycle, for example, by identifying the end of the T-wave. Further, the end-systolic right ventricle volume may be determined by identifying a maximum impedance in a time window between to successive QRS complexes, for example, between two successive R-waves, wherein the maximum impedance substantially reflects the end-systolic volume.

The impedance processing unit is adapted to process the impedance signals to determine an impedance parameter substantially reflecting the diastolic right ventricle volume and/or the right atrial volume. As above, the impedance signals may be synchronized with ECG/IEGM signals. The measurement of the impedance at the end-systolic right ventricle volume can be combined with measurements of impedances reflecting or related to the diastolic right ventricle volume and/or the right atrial volume.

The present invention is preferably implemented in an implantable heart stimulator such as a pacemaker e.g. a biventricular pacemaker. However, the invention is also applicable to other types of cardiac stimulators such as dual chamber stimulators, implantable cardioverter defibrillators (ICDs), etc.

According to the present invention, the impedance signals are processed to determine a second impedance parameter substantially reflecting a difference between diastolic and systolic volume. In a further alternative, the impedance signals are processed to determine a second impedance parameter substantially reflecting a right ventricular ejection-fraction.

Thus, according to the present invention, the first impedance parameter, which decreases with increasing right ventricle volume and left atrial pressure, and the second impedance parameter, which decreases with decreasing difference between diastolic and systolic volume right ventricle ejection fraction, are monitored over time for early detection of impaired left ventricle or right ventricle function. If both the first and second impedance parameters have decreased during a predetermined period of time, this is a strong indication of a worsening heart failure condition. This is caused by dilatation or blood accumulation in the right side of the heart. By using both the first and second impedance parameters in the monitoring, the specificity of the HF detection can be significantly improved. In fact, there are a number of situations where the right ventricle volume increases or where the peak to peak amplitude decreases, which is not caused by heart failure. These situations may be identified by monitoring both the first impedance parameter and the second impedance parameter and determining the heart failure status based on the development of both these parameters. A non exhaustive number of examples of such situations or conditions includes:
- Asynchronous ventricle contraction: The peak to peak amplitude may increase since larger ventricle movements are required in order to maintain the stroke volume.
- Too short filling time (AV interval): The stroke volume is reduced and the sinus rhythm is increased, which result in a reduced peak to peak amplitude with an unchanged or even increased right ventricle volume.
- Diving: At high ambient pressure, the oxygenation of the blood is increased and the cardiac frequency is reduced, resulting in an increased filling of the ventricles and an increased ejection fraction. This entails an increased peak to peak amplitude. At the same time, the ventricle volume is reduced and the impedance measured over the ventricles decreases.

In a further embodiment of the present invention, a rate of change of the first and the second impedance parameter are calculated for each cardiac cycle or for a number of cardiac cycles, and a heart failure status of the patient is determined to be deteriorating if a rate of change of a decrease of the first impedance parameter is higher than a predetermined threshold and a rate of change of a decrease of the second impedance parameter is higher than a predetermined threshold. This indicates a fast change of the right ventricle congestion, i.e. increased left atrial pressure. Such a fast change may be an early indication of acute worsening, which may be further communicated to the patient and/or the hospital. As a response to such an indication, the therapy can be changed and hospitalization may thereby be avoided. In an alternative embodiment, two thresholds may be used, where the first indicates a fast change and a second, lower threshold may indicate a worsening which is not acute. Thereby, slow impairments or slow improvements can be followed, for example, at changed drug titration or pacing therapy. Furthermore, the rate of change of the first and the second impedance parameter may be calculated over longer period than a cardiac cycle of a few consecutive cardiac cycles, for example, for a period of a couple of days or weeks.

Pacing therapy for heart failure patients can be altered as changed AV-delay, PV-delay, or VV-delay. If several pacing sites are available using multiple electrode leads, the pacing therapy can be optimized or the optimal pacing site(s) can be made automatically using the first and second impedance parameters as input. After changing a timing combination of pacing location, the change of impedance is recorded and compared to the values prior to the change. If the first impedance parameter increases (i.e. indicating an improvement of the heart failure status), the new setting is favourable and may be stored as the new setting. This may be an iterative procedure to optimize the pacing therapy and/or pacing site.

In embodiments of the present invention, a first electrode configuration includes a first electrode located in the right atrium (e.g. a ring electrode), a second electrode located in the right ventricle (e.g. a ring electrode), a third electrode located in the right atrium (e.g. a tip electrode), and a fourth electrode located in the right ventricle (e.g. a tip electrode), and wherein a second alternative electrode configuration includes a first electrode (e.g. a ring electrode) and a second electrode (e.g. a tip electrode) located in the right ventricle. There are other conceivable alternative electrode configurations that provides impedance signals that substantially reflect volume changes of the right ventricle and/or the right atrium, for example four electrodes located in the right ventricle, e.g. three ring electrodes and one tip electrode, or one coil electrode, one ring electrode and one tip electrode.

According to further embodiments of the present invention, impedance signals are acquired during consecutive cardiac cycles, wherein each impedance signal is acquired/obtained in synchronization with a specific cardiac event in respective cardiac cycle, and wherein the impedance processing unit is adapted to process the received impedance signal to determine the first impedance parameter to be a mean impedance value of the impedance signals and the second impedance parameter to be a peak-to-peak value of the impedance signal between a cardiac event in two consecutive cardiac cycles.

In accordance with embodiments of the present invention, the acquisition of impedance signals is synchronized with the respiration cycle.

In accordance with embodiments of the present invention, the impedance signals are processed to calculate average impedance values from which the first and second impedance parameters are determined, wherein each average value is calculated over a predetermined number of cardiac cycles, or wherein the impedance processing unit is adapted to process the impedance signals to determine the first and second impedance parameter as average values over a predetermined number of cardiac cycles.

According to an embodiment of the present invention, a maximum impedance is identified in a time window between to successive QRS complexes, for example, between two successive R-waves, or between to successive T-waves and the first impedance parameter is determined to be the identified maximum impedance. This maximum impedance coincides with the time-point for the end-systolic volume, i.e. the minimum volume, and will thus substantially reflect the end-systolic volume or the minimum volume. The time window may further be set to 1/4T to 3/4T of the period T, where the period T may be set to the period of time between the successive R-waves.

In accordance with a further embodiment, the heart failure status is compared with a predetermined patient specific threshold and, if the heart failure status exceeds the patient specific threshold, a warning signal that triggers an alarm function is issued. Preferably, the threshold is programmable. The threshold will be different for different methods of determining the patient status and for predicting the HF status and will also be patient specific. Since the impedance is affected not only by the HF status but also by lead location, blood constituents, if the patient is on dialysis, diet, exercise, posture, etc, the impedance (and hence the index) variability will be different for different patients. The alarm may be sent to the physician of the patient via a communication system using a system for remote follow-up. The physician may call the patient for a visit, or the device may notify the patient that he or she should seek contact with the/a physician.

According to a further embodiment, the patient data is transferred to and displayed on an external device such as programmer during a follow-up and a trend of the patient's heart failure status can be reviewed by, for example, a physician.

In a further embodiment, the patient status is regularly transferred to and displayed on an external device such as a home monitoring device located at the patient's home, and/or a programmer device located at the health care provider of the patient.

As the skilled person realizes, steps of the methods according to the present invention, as well as preferred embodiments thereof, are suitable to realize as computer program or as a computer readable medium.

Further objects and advantages of the present invention will be discussed below by means of exemplifying embodiments.

### Brief description of the drawings

Exemplifying embodiments of the invention will be described below with reference to the accompanying drawings, in which:
Fig. 1 is a simplified, schematic diagram illustrating generally one example of an implantable medical device including a heart failure detector in accordance with the present invention and an environment in which it is used;
Fig. 2 is schematic block diagram illustrating an embodiment of the implantable medical device in accordance with the present invention.
Fig. 3 is a schematic flow chart showing the principles of the method for detecting incipient heat failure.
Fig. 4a is a simplified, schematic diagram illustrating generally one example of an electrode configuration which may be used in the present invention.
Fig. 4b is a simplified, schematic diagram illustrating generally another example of an electrode configuration which may be used in the present invention.
Fig. 5 is a simplified, schematic diagram showing measured IEGM signals synchronized with acquired impedance signals during successive cardiac cycles.

### Description of exemplifying embodiments

The following is a description of exemplifying embodiments in accordance with the present invention. This description is not to be taken in limiting sense, but is made merely for the purposes of describing the general principles of the invention. Thus, even though particular types of implantable medical devices such as heart stimulators will be described, the invention is also applicable to other types of cardiac stimulators such as dual chamber stimulators, implantable cardioverter defibrillators (ICDs), etc.

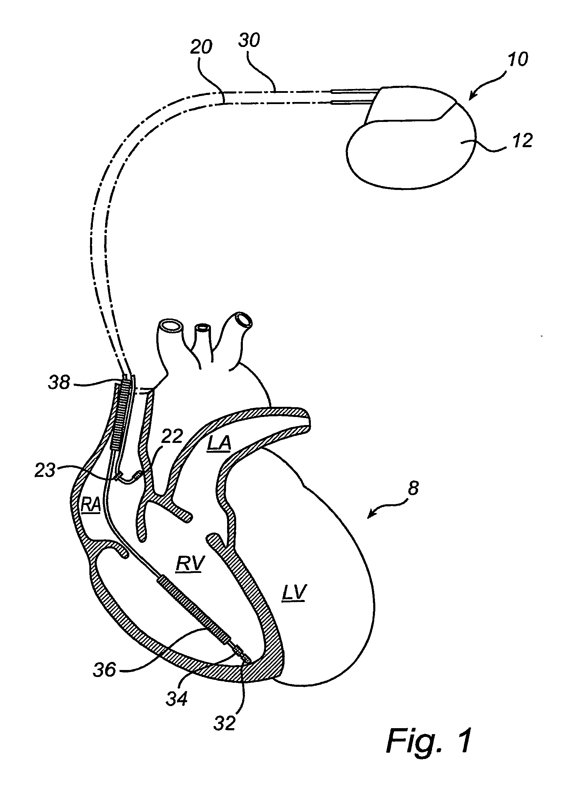

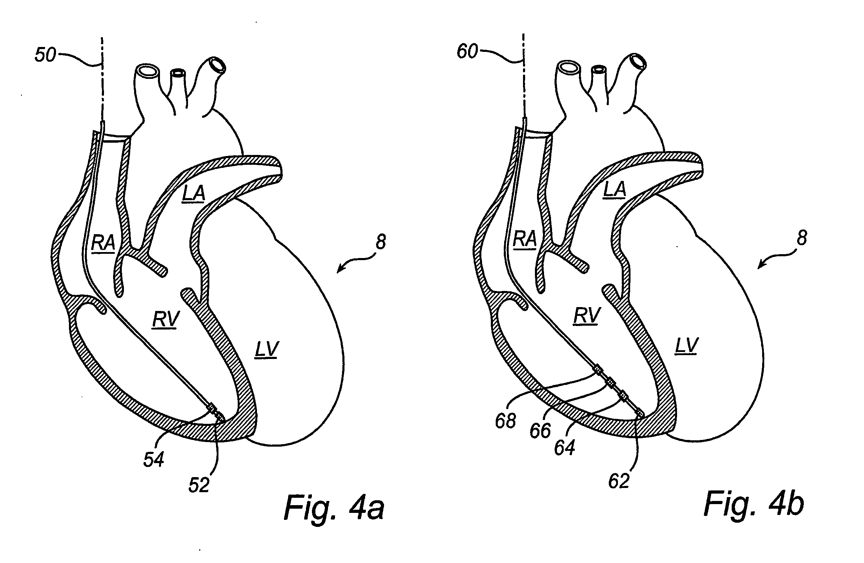

Fig. 1 simplified, schematic diagram illustrating generally one example of an implantable medical device 10 including a heart failure detector in accordance with the present invention and an environment in which it is used, including a heart 8. In this illustrated example, the implantable medical device 10 is an implantable pacemaker and the device 10 is in electrical communication with a patient's heart 8 by way of a right atrial lead 20 having a right atrial (RA) tip electrode 22 and an RA ring electrode 23 implanted in the atrial appendage. Further, the pacemaker 10 is also in electrical communication with the heart 1 by way a right ventricular lead 30 having a right ventricular (RV) tip electrode 32, an RV ring electrode 34, RV coil electrode 36, and a superior vena cava (SVC) coil electrode 38. Typically, the RV lead is transvenously inserted into the heart 8 so as to place the RV coil electrode 36 in the right ventricular apex and the SVC coil electrode 38 in the superior vena cava. Accordingly, the right ventricular lead 30 is capable of receiving cardiac signals, and delivering stimulation in the form of pacing to the right ventricle RV. In order to sense left atrial and ventricular cardiac signals and to provide left chamber pacing therapy, the pacemaker 10 may further be coupled to a "coronary sinus" lead (not shown) designed for placement in the coronary sinus region via the coronary sinus for positioning a distal electrode adjacent to the left atrium. As used herein, the wording "coronary sinus region" refers to the vasculature of the left ventricle, including any portion of the coronary sinus, great cardiac vein, left marginal vein, middle cardiac vein, and/or small cardiac vein or any other cardiac vein accessible by the coronary sinus. This particular electrode configuration of Fig. 1 is useful for providing conceptual clarity, however, other electrode configurations will be discussed further below. With reference to the configuration shown in Fig. 1, a number of measurement vectors that can be used for impedance measurements, for example, a current may be applied between the RV ring electrode 34 and the RA ring electrode 23 and the resulting voltage may be measured between the RV tip electrode 32 and the RA tip electrode 22. Although this configuration has been shown to provide good measurement results, it is understood that other configurations can be used and that the present invention is not limited to this configuration. In connection with Figs. 4a and 4b further measurement vectors will be discussed below.

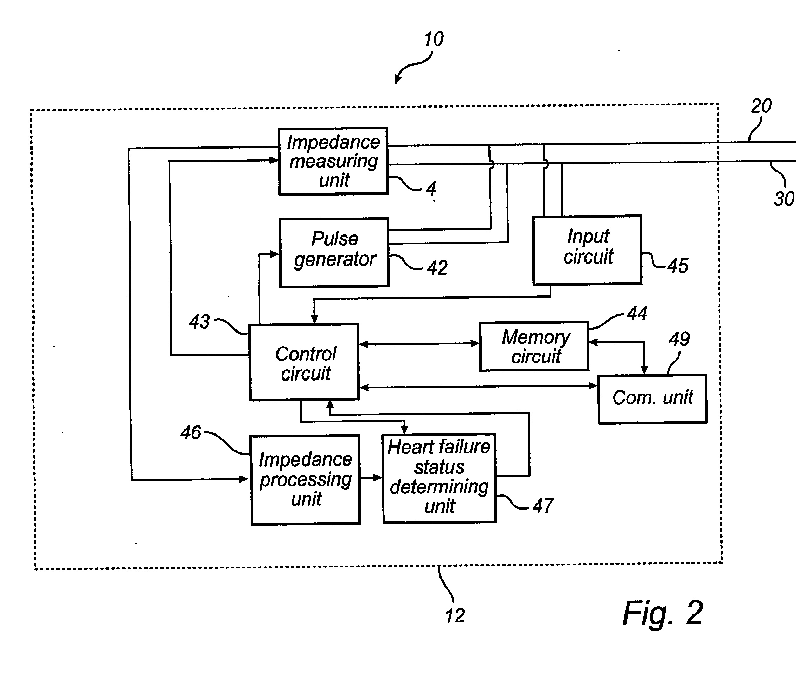

Turning now to Fig. 2, the pacemaker 10 of Fig. 1 is shown in a block diagram form. For illustrative purposes, reference is made to Fig. 1 for the elements of the leads that are intended for positioning in or at the heart. The heart stimulator 10 comprises a housing 12 being hermetically sealed and biologically inert, see Fig. 1. Normally, the housing is conductive and may, thus, serve as an electrode. One or more pacemaker leads, where two are shown in Fig. 1, 20, 30, are electrically coupled to the implantable medical device 10 in a conventional manner. The leads 20, 30 extend into the heart 8 (see Fig. 1).

As discussed above with reference to Fig. 1, the leads 20, 30 each comprises one or more electrodes, such coils, tip electrodes or ring electrodes, arranged to, inter alia, transmit pacing pulses for causing depolarization of cardiac tissue adjacent to the electrode(-s) generated by a pace pulse generator 42 under influence of a control circuit 43 comprising a microprocessor, and for measuring impedances reflecting the septal wall movements. The control circuit 43 controls, inter alia, pace pulse parameters such as output voltage and pulse duration. A memory circuit 44 is connected to the control circuit 43, which memory circuit 44 may include a random access memory (RAM) and/or a non-volatile memory such as a read-only memory (ROM). Detected signals from the patients heart are processed in an input circuit 45 and are forwarded to the microprocessor of the control circuit 43 for use in logic timing determination in known manner. The stimulator also includes a communication unit 49, for example, a telemetry unit or RF transceiver adapted for bi-directional communication with external devices. Furthermore, an impedance measuring unit 41 for measuring at least an impedance indicative of the right ventricular volume (e.g. between the RV electrodes 32, 34 and the RA electrodes 22, 23). The impedance is modulated as the right ventricle and the right atrium contract and expand. An input circuit 45 including sense amplifiers and signal processing circuitry is connected to the leads 20, 30 is adapted to, inter alia ,detect intrinsic electrical heart depolarizations to detect QRS complexes, which are depolarizations corresponding to ventricular heart contractions. The time interval between two successive QRS complexes can be used to define a cardiac cycle, for example, the time interval between the R-wave or T-wave of two succesive QRS complexes can be used to define a cardiac cycle. According to examples of the present invention, a peak to peak value (i.e. the difference between the maximum impedance and the minimum impedance) is determined for each cardiac cycle for use in the heart failure monitoring. In another example, a mean impedance value is determined over a number of consecutive cardiac cycles.

The impedance measuring unit 41 is adapted to carry out impedance measurements of the intracardiac and intrathoracic impedance or admittance of the patient, for example, by applying a current between the RV ring electrode 34 and the RA ring electrode 23 and the measuring the resulting voltage between the RV tip electrode 32 and the RA tip electrode 22. The raw impedance data is then supplied to an impedance processing module 46, which may comprise, for example, amplifiers and filters e.g. FIR or IIR filters. The impedance processing unit 46 performs a pre-processing procedure in which the acquired impedance or admittance signal are pre-processed. According to the present invention, the impedance processing unit 46 determines a first impedance parameter that substantially reflects volume changes of the right ventricle. This first impedance parameter may be determined by low-pass filtering the raw impedance signal, e.g. about 0 to about 5 Hz or about 0 to about 2.5 Hz, and averaging the filtered signal between two detected heart events, for example, two R-waves or two P-waves, which results in one value for each cardiac cycle. In embodiments of the present invention, the resistive part of the impedance is used when determining the impedance parameters.

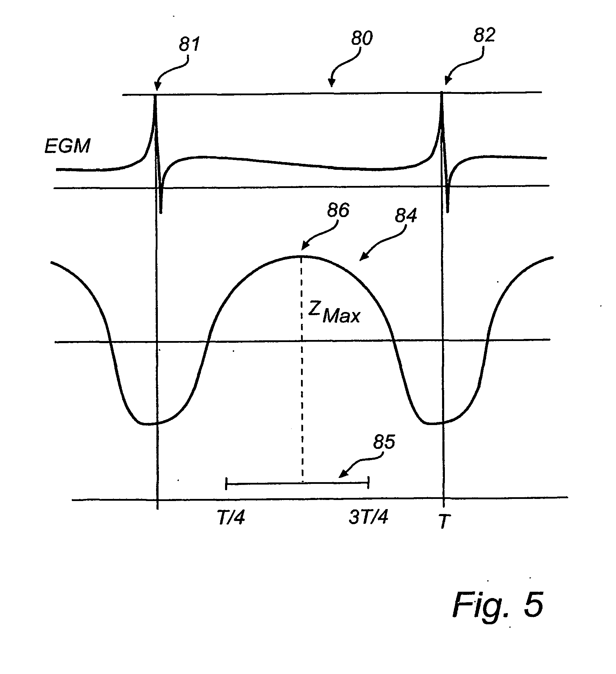

In one embodiment of the present invention, the first impedance parameter is processed such that is substantially reflects the end-systolic right ventricle volume. The acquired impedance signals (the resistive part of the low-pass filtered signal) may be synchronized with IEGM signals in order to locate the end-systolic right ventricle volume. A maximum impedance may identified in a time window between to successive QRS complexes, for example, between two successive R-waves, or between to successive T-waves and the first impedance parameter may be determined to be the identified maximum impedance. This maximum impedance coincides with the time-point for the end-systolic volume, i.e. the minimum volume, and will thus substantially reflect the end-systolic volume or the minimum volume. The time window may further be set to 1/4T to 3/4T of the period T, where the period T may be set to the period of time between the successive R-waves. In Fig. 5, obtained IEGM signals 80 including two successive QRS complexes 81 and 82 synchronized in time with impedance signals 84, for example, obtained in the right ventricle. A time window 85 is set between the two successive QRS complexes 81 and 82, respectively, and the maximum impedance 86 is identified. The interval T between the QRS complexes 81 and 82 may be defined as the time interval between the R-wave of respective QRS complex and the time window, during which the search for maximum impedance is performed, may be set of 1/4T to 3/4T.

Further, in other embodiments, the first impedance parameter substantially reflects the diastolic right ventricle volume and/or the right atrial volume. The first impedance parameter may be determined to reflect a combination of the end-systolic right ventricle volume and/or the diastolic right ventricle volume and/or the right atrial volume.

Moreover, the impedance processing unit 46 is adapted to determine a second impedance parameter corresponding to a peak to peak value between a maximum impedance and a minimum impedance. The second impedance parameter may be determined by band-pass filtering the raw impedance signal, e.g. about 0.2 to about 100 Hz or about 0.3 to about 65 Hz, and determining the peak to peak amplitude of the band-pass filtered signal between two heart events, which results in one value for each cardiac cycle. In order to reduce the noise, the data acquisition can be synchronized with the respiration frequency, for example, by using cardiac cycles or heart beat from the same relative position in the respiration cycle. Another way to reduce the respiration artefacts, is to determine each impedance parameter (or to use raw impedance data) as an average value over at least two full respiratory cycles.

The impedance parameter, the first and second parameter, are supplied to a heart failure status determining module 47 adapted to determine a patient status. Thus, the patients' heart failure status can be monitored and tracked and/or acute heart failure exacerbation events in the patient can be predicted, which will be described in more detail hereinafter.

As discussed above, the first impedance parameter decreases with increasing right ventricle volume and left atrial pressure. The second impedance parameter decreases with decreasing difference between diastolic and systolic volume and with decreasing ejection fraction. When the right ventricle dilates as an effect of e.g. increased right ventricle pressure, the wall tension increases which reduces the compliance of the walls. The ventricle becomes more spherical to distribute the tension more evenly. Also, if the stroke volume is unchanged and the right ventricle volume increases, the relative difference between diastolic and systolic volumes will decrease.

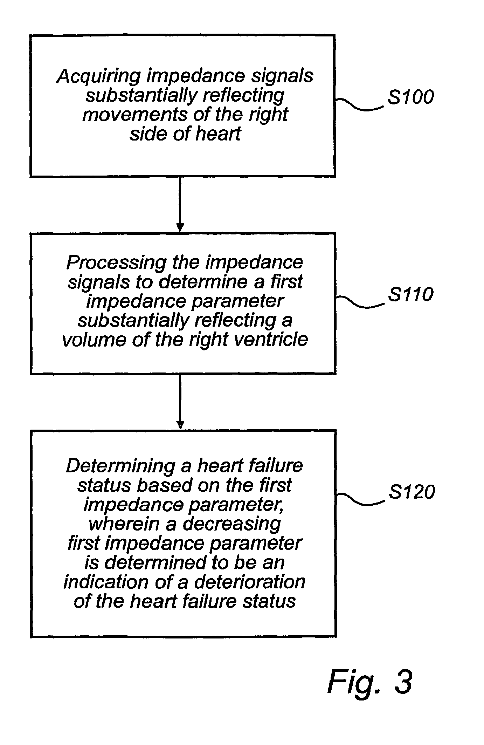

Turning now to Fig. 3, an embodiment of the general concept of method will be discussed. First, at step S100, impedance signals reflecting volume changes of the right ventricle of a heart are acquired, for example, by applying a current between the RV ring electrode 34 and the RA ring electrode 23 and the measuring the resulting voltage between the RV tip electrode 32 and the RA tip electrode 22. The impedance signals may be pre-processed as described above including amplifying and filtering. Thereafter, at step S110, the impedance signals are processed to determine a first impedance parameter substantially reflecting a volume of the right ventricle. Then, at step S120, a heart failure status is determined based on the first impedance parameter, wherein a decreasing first impedance parameter is determined to be an indication of a deterioration of the heart failure status. For example, if the first parameter has decreased during a predetermined period of time, e.g. a couple of days, or if the first parameter has decreased below a predetermined threshold, this may be an indication of a deterioration. The conditions or rules used to determine when a decrease is an indication of a deterioration of the heart failure status may be patient specific and may be adaptable. For example, a patient specific threshold value be determined during a calibration period following the implantation and may be recalibrated if necessary.

With reference now to Figs. 4a and 4b, further conceivable electrode configurations will be discussed, respectively. In Fig. 4a, a bi-polar electrode configuration is shown. A right ventricular lead 50 having a right ventricular (RV) tip electrode 52 and a RV ring electrode 54 is connected to an implantable medical device (see Fig. 1 and 2) including a heart failure detector in accordance with the present invention. In this illustrated example, the implantable medical device is an implantable pacemaker as described above in connection with Figs 1 and 2, and the device is in electrical communication with a patient's heart 8 by way of the lead 50 and electrodes 52 and 54. Using this electrode configuration, a current may be applied between the electrodes 52 and 54, the resulting voltage may be measured between the same electrode and the impedance calculated. In this case, the obtained impedance signals will mainly reflect the volume changes of the right ventricle.

In Fig. 4b, a quadri-polar electrode configuration is shown. A right ventricular lead 60 having a right ventricular (RV) tip electrode 62, and RV ring electrodes 64, 66, 68 is connected to an implantable medical device (see Fig. 1 and 2) including a heart failure detector in accordance with the present invention. In this illustrated example, the implantable medical device is an implantable pacemaker as described above in connection with Figs 1 and 2, and the device is in electrical communication with a patient's heart 8 by way of the lead 60 and electrodes 62 and 64, 66, 68, respectively. Using this electrode configuration, a current may be applied between the ring electrodes 64 and 66, respectively, and the resulting voltage may be measured between the tip electrode 62 and the ring electrode 68. In this case, the obtained impedance signals will mainly reflect both the volume changes of the right ventricle. Furthermore, there are other conceivable electrode configurations (see Fig. 1), for example, a tri-polar configuration where a right ventricle lead having one tip electrode, one ring electrode and one coil electrode is used.

Although an exemplary embodiment of the present invention has been shown and described, it will be apparent to those having ordinary skill in the art that a number of changes, modifications, or alterations to the inventions as described herein may be made.

## Claims

1. A heart failure detector (10) for detecting incipient heart failure of a patient comprising:
an impedance measuring unit (4) adapted to obtain impedance signals reflecting volume changes of the right ventricle of a heart (8) of said patient, said impedance measuring unit (4) being connectable to at least two electrodes (22, 23, 32, 34, 36, 38, 52, 54, 62, 64, 66, 68) adapted to be located in said right ventricle such that volume changes of said right ventricle and/or said right atrium are reflected by said impedance signals;
an impedance processing unit (46) adapted to receive said impedance signals from said impedance measuring unit (4) and to
i) process said impedance signals to determine a first impedance parameter substantially reflecting a volume of the right ventricle;
ii) process said impedance signals to determine a second impedance parameter substantially reflecting a difference between diastolic and systolic volume or substantially reflecting a right ventricular ejection-fraction; and
a heart failure status determining unit (47) adapted to determine a heart failure status of said patient based on said first and said second impedance parameters, **characterized in that**
said heart failure status determining unit (47) is adapted to determine that said heart failure status of said patient is deteriorating if said first impedance parameter has decreased below a predetermined first impedance parameter threshold, and said second impedance parameter has decreased, or if said second impedance parameter remains substantially unchanged.

2. The heart failure detector according to claim 1, further including a QRS detector circuit (45) connectable to said electrodes (22, 23, 32, 34, 36, 38, 52, 53, 62, 64, 66, 68), said QRS detector (45) is adapted to detect the occurrence of cardiac events in the cardiac cycle, and wherein said impedance processing unit (46) is adapted to synchronize detected cardiac events with said impedance signals and to process said impedance signals such that the first impedance parameter reflects an end-systolic volume of the right ventricle.

3. The heart failure detector according to claim 2, wherein
said QRS detector (45) is adapted to identify cardiac events in successive QRS complexes; and
said impedance processing unit (46) is adapted to identify a maximum impedance in a time window between two successive QRS complexes, wherein said maximum impedance substantially reflects the end-systolic volume, and to determine said first impedance parameter to be said identified maximum impedance.

4. The heart failure detector according to any one of preceding claims, wherein
said impedance processing unit (46) is adapted to calculate a rate of change of said first and said second impedance parameter for each cardiac cycle or for a number of cardiac cycles, and wherein
said heart failure status determining unit (47) is adapted to determine that a heart failure status of said patient is deteriorating if a rate of change of a decrease of said first impedance parameter is higher than a predetermined threshold, and/or a rate of change of a decrease of said second impedance parameter is higher than a predetermined threshold.

5. The heart failure detector according to any one of preceding claims, wherein said at least two electrodes (22, 23, 32, 34, 36, 38, 52, 54, 62, 64, 66, 68) are adapted to be implanted within or at the right ventricle and/or right atrium of said heart (8), wherein a first electrode configuration includes a first electrode (22) located in the right atrium, a second electrode (32) located in the right ventricle, a third electrode (23) located in the right atrium, and a fourth electrode (34) located in the right ventricle, and wherein a second electrode configuration includes a first electrode (52, 62) and a second electrode (54, 64, 66, 68) both located in the right ventricle.

6. The heart failure detector according to any one of preceding claims, wherein said impedance measuring unit (4) is adapted to obtain said impedance signals during consecutive cardiac cycles, wherein each impedance signal is obtained in synchronization with a specific cardiac event in respective cardiac cycle, and wherein said impedance processing unit (46) is adapted to process said received impedance signal to determine said first impedance parameter to be a mean impedance value of the impedance signals and said second impedance parameter to be a peak-to-peak value of the impedance signal between a cardiac event in two consecutive cardiac cycles.

## Patentansprüche

1. Detektor für Herzversagen (10) zum Detektieren von beginnendem Herzversagen eines Patienten, umfassend:
eine Impedanzmesseinheit (4), die dafür geeignet ist, Impedanzsignale zu erhalten, welche Volumenänderungen der rechten Herzkammer (8) des Patienten wiedergeben, wobei die Impedanzmesseinheit (4) mit mindestens zwei Elektroden (22, 23, 32, 34, 36, 38, 52, 54, 62, 64, 66, 68) verbunden werden kann, die dafür geeignet sind, derart in der rechten Kammer angeordnet zu sein, dass Volumenänderungen der rechten Kammer und/oder des rechten Vorhofs von den Impedanzsignalen wiedergegeben werden;
eine Impedanzverarbeitungseinheit (46), die dafür geeignet ist, Impedanzsignale von der Impedanzmesseinheit (4) zu empfangen, und
(i) die Impedanzsignale zu verarbeiten, um einen ersten Impedanzparameter zu bestimmen, welcher im Wesentlichen ein Volumen der rechten Kammer wiedergibt,;
(ii) die Impedanzsignale zu verarbeiten, um einen zweiten Impedanzparameter zu bestimmen, der im Wesentlichen eine Differenz zwischen diastolischem und systolischem Volumen wiedergibt oder im Wesentlichen eine rechtsventrikuläre Ausstoßfraktion wiedergibt; und
eine Einheit zum Bestimmung des Status des Herzversagens (47), die dafür geeignet ist, einen Status des Herzversagens des Patienten auf der Grundlage des ersten und des zweiten Impedanzparameters zu bestimmen, **dadurch gekennzeichnet, dass**
die Einheit zum Bestimmung des Status des Herzversagens (47) dafür geeignet ist festzustellen, dass sich der Status des Herzversagens des Patienten verschlechtert, wenn sich der erste Impedanzparameter unter einen im Voraus festgelegten ersten Impedanzparameter-Schwellenwert verringert hat und sich der zweite Impedanzparameter verringert hat oder wenn der zweite Impedanzparameter im Wesentlichen unverändert bleibt.

2. Detektor für Herzversagen nach Anspruch 1, der ferner eine QRS-Detektorschaltung (45) enthält, die mit den Elektroden (22, 23, 32, 34, 36, 38, 52, 53, 62, 64, 66, 68) verbunden werden kann, wobei der QRS-Detektor (45) dafür geeignet ist, das Auftreten kardialer Ereignisse im kardialen Zyklus festzustellen, und wobei die Impedanzverarbeitungseinheit (46) dafür geeignet ist, festgestellte kardiale Ereignisse mit den Impedanzsignalen zu synchronisieren und die Impedanzsignale derart zu verarbeiten, dass der erste Impedanzparameter ein endsystolisches Volumen der rechten Kammer wiedergibt.

3. Detektor für Herzversagen nach Anspruch 2, wobei
der QRS-Detektor (45) dafür geeignet ist, kardiale Ereignisse in aufeinanderfolgenden QRS-Komplexen zu identifizieren; und
die Impedanzverarbeitungseinheit (46) dafür geignet ist, in einem Zeitfenster zwischen zwei aufeinanderfolgenden QRS-Komplexen eine maximale Impedanz zu identifizieren, wobei die maximale Impedanz im Wesentlichen das endsystolische Volumen wiedergibt, und um festzustellen, dass der erste Impedanzparameter der identifizierten maximalen Impedanz entspricht.

4. Detektor für Herzversagen nach einem der vorhergehenden Ansprüche, wobei
die Impedanzverarbeitungseinheit (46) dafür geeignet ist, eine Rate der Veränderung des ersten und des zweiten Impedanzparameters für jeden kardialen Zyklus oder für eine Reihe von kardialen Zyklen zu berechnen, und wobei
die Einheit zum Bestimmen des Status des Herzversagens (47) dafür geeignet ist festzustellen, dass sich ein Status des Herzversagens des Patienten verschlechtert, wenn eine Rate der Veränderung einer Verringerung des ersten Impedanzparameters höher als ein im Voraus festgelegter Schwellenwert ist und/oder eine Rate der Veränderung einer Verringerung des zweiten Impedanzparameters höher als ein im Voraus festgelegter Schwellenwert ist.

5. Detektor für Herzversagen nach einem der vorhergehenden Ansprüche, wobei die mindestens zwei Elektroden (22, 23, 32, 34, 36, 38, 52, 54, 62, 64, 66, 68) dafür geeignet sind, in oder an die rechte Kammer und/oder den rechten Vorhof des Herzens (8) implantiert zu werden, wobei eine erste Elektrodenkonfiguration eine sich im rechten Vorhof befindende erste Elektrode (22), eine sich in der rechten Kammer befindende zweite Elektrode (32), eine sich im rechten Vorhof befindende dritte Elektrode (23) und eine sich in der rechten Kammer befindende vierte Elektrode (34) umfasst, und wobei eine zweite Elektrodenkonfiguration eine erste Elektrode (52, 62) und eine zweite Elektrode (54, 64, 66, 68) umfasst, die sich beide in der rechten Kammer befinden.

6. Detektor für Herzversagen nach einem der vorhergehenden Ansprüche, wobei die Impedanzmesseinheit (4) dafür geeignet ist, die Impedanzsignale während aufeinanderfolgender kardialer Zyklen zu erhalten, wobei jedes Impedanzsignal in Synchronisation mit einem bestimmten kardialen Ereignis im jeweiligen kardialen Zyklus erhalten wird, und wobei die Impedanzverarbeitungseinheit (46) dafür geeignet ist, das empfangene Impedanzsignal zu verarbeiten, um festzustellen, dass es sich bei dem ersten Impedanzparameter um einen mittleren Impedanzwert der Impedanzsignale handelt und es sich bei dem zweiten Impedanzparameter um einen Spitze-zu-Spitze-Wert des Impedanzsignals zwischen einem kardialen Ereignis in zwei aufeinanderfolgenden kardialen Zyklen handelt.

## Revendications

1. Détecteur d'insuffisance cardiaque (10) destiné à détecter une insuffisance cardiaque débutante chez un patient, comprenant :
une unité de mesure d'impédance (4), conçue pour obtenir des signaux d'impédance reflétant des modifications de volume du ventricule droit du coeur (8) dudit patient, ladite unité de mesure d'impédance (4) étant raccordable à au moins deux électrodes (22, 23, 32, 34, 36, 38, 52, 54, 62, 64, 66, 68) conçues pour être disposées dans ledit ventricule droit de manière à ce que des modifications de volume dudit ventricule droit et/ou de ladite oreillette droite soient reflétées par lesdits signaux d'impédance ;
une unité de traitement d'impédance (46) conçue pour recevoir lesdits signaux d'impédance provenant de ladite unité de mesure d'impédance (4) et pour
i) traiter lesdits signaux d'impédance pour déterminer un premier paramètre d'impédance reflétant essentiellement un volume du ventricule droit ;
ii) traiter lesdits signaux d'impédance pour déterminer un second paramètre d'impédance reflétant essentiellement une différence entre le volume diastolique et systolique ou reflétant essentiellement une fraction d'éjection ventriculaire droite ; et
une unité de détermination du statut d'insuffisance cardiaque (47) conçue pour déterminer un statut d'insuffisance cardiaque dudit patient à partir dudit premier et dudit second paramètre d'impédance, **caractérisé en ce que**
ladite unité de détermination du statut d'insuffisance cardiaque (47) est conçue pour déterminer que ledit statut d'insuffisance cardiaque dudit patient se détériore si ledit premier paramètre d'impédance a diminué en dessous d'un seuil prédéterminé du premier paramètre d'impédance et ledit second paramètre d'impédance a diminué ou si ledit second paramètre d'impédance reste essentiellement inchangé.

2. Détecteur d'insuffisance cardiaque selon la revendication 1, comprenant également un circuit de détecteur QRS (45) raccordable auxdites électrodes (22, 23, 32, 34, 36, 38, 52, 53, 62, 64, 66, 68), ledit détecteur QRS (45) étant conçu pour détecter l'occurrence d'évènements cardiaques dans le cycle cardiaque, et dans lequel ladite unité de traitement d'impédance (46) est conçue pour synchroniser des évènements cardiaques détectés avec lesdits signaux d'impédance et pour traiter lesdits signaux d'impédance de manière à ce que le premier paramètre d'impédance reflète un volume télésystolique du ventricule droit.

3. Détecteur d'insuffisance cardiaque selon la revendication 2, dans lequel ledit détecteur QRS (45) est conçu pour identifier des évènements cardiaques dans des complexes QRS successifs ; et ladite unité de traitement d'impédance (46) est conçue pour identifier une impédance maximale dans une fenêtre temporelle entre deux complexes QRS successifs, ladite impédance maximale reflétant essentiellement le volume télésystolique, et pour déterminer ledit premier paramètre d'impédance comme étant ladite impédance maximale identifiée.

4. Détecteur d'insuffisance cardiaque selon l'une quelconque des revendications précédentes, dans lequel ladite unité de traitement d'impédance (46) est conçue pour calculer un taux de modification dudit premier et dudit second paramètre d'impédance pour chaque cycle cardiaque ou pour un certain nombre de cycles cardiaques, et dans lequel
ladite unité de détermination du statut d'insuffisance cardiaque (47) est conçue pour déterminer qu'un statut d'insuffisance cardiaque dudit patient se détériore si un taux de modification d'une diminution dudit premier paramètre d'impédance est supérieur à un seuil prédéterminé et/ou si un taux de modification d'une diminution dudit second paramètre d'impédance est supérieur à un seuil prédéterminé.

5. Détecteur d'insuffisance cardiaque selon l'une quelconque des revendications précédentes, dans lequel lesdites deux électrodes ou plus (22, 23, 32, 34, 36, 38, 52, 54, 62, 64, 66, 68) sont conçues pour être implantées dans ou au niveau du ventricule droit et/ou de l'oreillette droite dudit coeur (8), une première configuration d'électrodes comprenant une première électrode (22) disposée dans l'oreillette droite, une deuxième électrode (32) disposée dans le ventricule droit, une troisième électrode (23) disposée dans l'oreillette droite et une quatrième électrode (34) disposée dans le ventricule droit, et une deuxième configuration d'électrodes comprenant une première électrode (52, 62) et une seconde électrode (54, 64, 66, 68), toutes les deux disposées dans le ventricule droit.

6. Détecteur d'insuffisance cardiaque selon l'une quelconque des revendications précédentes, dans lequel ladite unité de mesure d'impédance (4) est conçue pour obtenir lesdits signaux d'impédance pendant des cycles cardiaques consécutifs, dans lequel chaque signal d'impédance est obtenu en synchronisation avec un évènement cardiaque spécifique dans un cycle cardiaque respectif et dans lequel ladite unité de traitement d'impédance (46) est conçue pour traiter ledit signal d'impédance reçu pour déterminer ledit premier paramètre d'impédance comme étant une valeur d'impédance moyenne des signaux d'impédance et ledit second paramètre d'impédance comme étant une valeur crête-à-crête du signal d'impédance entre un évènement cardiaque dans deux cycles cardiaques consécutifs.
